Europäisches Patentamt

European Patent Office

Office européen des brevets

(11) Publication number: **0 379 903 B1**

(12) **EUROPEAN PATENT SPECIFICATION**

(45) Date of publication of patent specification: **31.08.94**

(21) Application number: **90100549.6**

(22) Date of filing: **11.01.90**

(51) Int. Cl.⁵: **C12P 13/04**, C12N 1/20,
//(C12N1/20,C12R1:13,1:15),
(C12P13/04,C12R1:13,1:15)

(54) **Process for producing L-amino acids by fermentation.**

(30) Priority: **13.01.89 JP 6955/89**

(43) Date of publication of application:
**01.08.90 Bulletin 90/31**

(45) Publication of the grant of the patent:
**31.08.94 Bulletin 94/35**

(84) Designated Contracting States:
**BE DE FR GB IT**

(56) References cited:
**FR-A- 2 491 495
US-A- 3 878 044
US-A- 3 929 571
US-A- 4 656 135**

**PATENT ABSTRACTS OF JAPAN, vol. 3, no.
72 (C-49), 21 June 1979; p. 17 C 49&NUM;**

**CHEMICAL ABSTRACTS, vol. 82, 1975, Columbus, OH (US); p. 409, no. 153757v&NUM;**

(73) Proprietor: **AJINOMOTO CO., INC.
5-8, Kyobashi 1-chome, Chuo-ku
Tokyo 104 (JP)**

(72) Inventor: **Tsuchida, Takayasu, c/o Ajinomoto
Co.Inc.
Kawasaki Plant,
No. 1-1, Suzuki-cho,
Kawasaki-ku
Kawasaki-shi, Kanagawa-ken (JP)**
Inventor: **Uchibori, Haruo, c/o Ajinomoto
Co.Inc.
Kawasaki Plant,
No. 1-1, Suzuki-cho,
Kawasaki-ku
Kawasaki-shi, Kanagawa-ken (JP)**
Inventor: **Takeuchi, Hiroshi, c/o Ajinomoto
Co.Inc.
Kawasaki Plant,
No. 1-1, Suzuki-cho,
Kawasaki-ku
Kawasaki-shi, Kanagawa-ken (JP)**
Inventor: **Seki, Mitsuyoshi, c/o Ajinomoto
Co.Inc.
Kawasaki Plant,
No. 1-1, Suzuki-cho,
Kawasaki-ku
Kawasaki-shi, Kanagawa-ken (JP)**

Rank Xerox (UK) Business Services
(3. 10/3.0 9/3.3.3)

(74) Representative: **Strehl Schübel-Hopf Groening & Partner**
**Maximilianstrasse 54**
**D-80538 München (DE)**

**Description**

The present invention relates to a process for producing L-amino acids by fermentation and to microorganisms useful in this process. L-Amino acids have been widely used as seasonings, medical drugs, feed additives, chemicals, reagents and the like.

L-amino acids produced by fermentation in an industrial scale are L-glutamic acid, L-lysine, L-glutamine, L-arginine, L-phenylalanine, L-alanine, L-threonine, L-isoleucine, L-histidine, L-proline, L-valine, L-serine, L-ornithine, L-citrulline, L-tyrosine, L-tryptophan and L-leucine, etc. Examples of microorganisms utilized for their production are those belonging to the genus Brevibacterium, the genus Corynebacterium, the genus Bacillus, the genus Escherichia, the genus Serratia, the genus Providencia, the genus Arthrobacter, etc.

In order to industrially produce L-amino acids at low costs utilizing these various microorganisms, improved breeding of microorganism has often been used. Since the L-amino acid productivity of wild strains per se is extremely poor in many cases there have been applied methods to induce mutations. Known is a method for imparting nutrient auxotrophy, imparting analog resistance or imparting nutrient auxotrophy in combination with analog resistance by artificial mutation; or a method for potentiating a gene for amino acid biosynthesis, etc. by genetic recombination, and the like.

It is important to produce L-amino acids at low costs in an industrial scale by enhancing the fermentation yield and the accumulated amount of L-amino acids.

FR-A-2 491 495 describes a process for the preparation of amino acids in which a microorganism producing an amino acid and being capable of assimilating lactic acid is cultured under aerobic conditions in the presence of at least one microorganism being capable of assimilating a hydrocarbon and being capable of converting said hydrocarbon to lactic acid. The resulting amino acids are accumulated in the culture medium and recovered therefrom.

JP-A-54 44 096 discloses the preparation of L-arginine by the cultivation of bacteria belonging to the genus Brevibacterium, which are resistant to 2-thiazol and deficient for L-histidine, L-proline, L-threonine, L-isoleucine, L-methionine or L-tryptophane. After cultivation of the bacteria the L-arginine is isolated from the culture medium.

JP-A-74 118 891 describes the preparation of L-lysine by Corynebacterium mutants being resistant to S-2-aminoethyl-L-cystine and deficient in the histidine-, phenylalanine-, proline-, adenine-, guanine-, methionine-, and leucine-synthesis pathway. After culturing the bacterium L-lysine is recovered from the culture medium.

In order to further improve the fermentation yield of L-amino acids and to develop a process for producing L-amino acids by fermentation which is advantageous from the industrial point of view, the present inventors have made extensive investigations. As a result, it has been found for the first time that by breeding strains having resistance to a peptide containing glutamic acid or aspartic acid, the yield of L-amino acids can be improved. Based on this finding, further investigations have been made and as the result, the present invention has been attained.

The present invention provides a process for producing an L-amino acid which comprises culturing in a liquid medium an L-amino acid-producing microorganism belonging to the genus Brevibacterium or the genus Corynebacterium, accumulating said L-amino acid in the culture medium and collecting said L-amino acid from the culture medium, wherein the L-amino acid-producing microorganism has resistance to a peptide containing. glutamic acid or aspartic acid.

There is further provided a microorganism belonging to the genus Brevibacterium or the genus Corynebacterium which is Brevibacterium flavum AJ 12418 (FERM BP-2205), Corynebacterium acetoacidophilum AJ 12419 (FERM BP-2206), Brevibacterium lactofermentum AJ 12420 (FERM BP-2207), Corynebacterium glutamicum AJ 12421 (FERM BP-2208), Brevibacterium flavum AJ 12422 (FERM BP-2209), Brevibacterium lactofermentum AJ 12423 (FERM BP-2210), Corynebacterium glutamicum AJ 12424 (FERM BP-2211), Brevibacterium flavum AJ 12425 (FERM BP-2212), Corynebacterium glutamicum AJ 12426 (FERM BP-2213), Brevibacterium flavum AJ 12427 (FERM BP-2214), Brevibacterium flavum AJ 12428 ((FERM BP-2215).

Strains having resistance to at least one of them are referred to as peptide-resistant strains of the present invention.

The above peptide containing glutamic acid or aspartic acid is represented by X-Glu, Glu-X, X-Asp or Asp-X, wherein X is preferably

3

Gly, Ala, Val, Leu, Ile, Arg,
Asp, Cit, Cys, Lys, Glu, Gln, His, Met, Orn, Pro, OH-Pro,
Phe, Tyr, Trp, Ser or Thr.

The L-amino acid produced by the method of this invention is any amino acid producible by fermentation. Examples are L-glutamic acid, L-glutamine, L-lysine, L-arginine, L-phenylalanine, L-threonin, L-isoleucine, L-histidine, L-proline, L-valine, L-serine, L-ornithine, L-citrulline, L-tyrosine, L-tryptophan and L-leucine, etc. The present invention can apply even to L-amino acids other than those exemplified herein as long as they are L-amino acids which can be produced by fermentation.

The microorganism belonging to the genus Brevibacterium or the genus Corynebacterium which can be used in the present invention is a variant having the peptide resistance described above and capable of producing an L-amino acid.

For obtaining the mutant of the present invention, the peptide resistance described above may be induced into parent strains shown below; alternatively, the peptide resistance may also be induced into mutants capable of producing L-amino acid.

Wild strains which can be parent strains of the mutants of the present invention are bacteria belonging to the genus Brevibacterium or the genus Corynebacterium known as Coryneform L-glutamic acid producing bacteria, and are exemplified by the following bacteria.

Brevibacterium flavum ATCC 14067
Brevibacterium lactofermentum ATCC 13869
Brevibacterium divaricatum ATCC 14020
Brevibacterium saccharolyticum ATCC 14066
Corynebacterium glutamicum ATCC 13032
Corynebacterium acetoacidophilum ATCC 13870

For the mutation treatment to produce mutants of the present invention from these parent strains, a conventional method such as a treatment with N-methyl-N'-nitro-N-nitrosoguanidine, etc. can be used. The isolation of the mutant of the present invention from the treated bacteria solution can be effected by screening and collecting strains which can grow in a medium containing the peptide.

A concrete mutation method to produce the variant of the present invention and the relationship between the peptide concentration and growth degree of the strain are shown below.

Mutation method

Bacteria cells of Brevibacterium flavum ATCC 14067, which had been grown in a bouillon agar slant at 30°C for 24 hours, were suspended in M/30 phosphate buffer solution in a cell density of $10^9$/ml. To the cell suspension was added 200 $\mu$g/ml of N-methyl-N'-nitro-N-nitrosoguanidine. The mixture was maintained at 0°C for 20 minutes followed by centrifugation. The cells were inoculated on a medium having the composition shown in Table 1 and cultured at 31.5°C for 2 to 10 days.

4

Table 1

| Composition of the Medium | |
|---|---|
| Component | Content |
| Glucose | 1.0 g/dl |
| Urea | 0.2 g/dl |
| $KH_2PO_4$ | 0.1 g/dl |
| $MgSO_4.7H_2O$ | 0.1 g/dl |
| $FeSO_4.7H_2O$ | 0.002 g/dl |
| $MnSO_4.7H_2O$ | 0.002 g/dl |
| Biotin | 100 $\mu$g/l |
| Thiamine hydrochloride | 100 $\mu$g/l |
| Tyr-Glu | 0.5 g/dl |
| Agar | 2.0 g/dl |
| | (pH 7.0) |

From 200 Tyr-Glu-resistant strains grown in the agar medium, Brevibacterium flavum AJ 12418 (FERM BP-2205) was obtained as a strain having high productivity of L-glutamine.

By procedures similar to the above mutation, strains having more improved productivity of amino acids could be obtained using various amino acid-producing strains as the original strain.

Representative examples are shown in Table 2.

In addition to the improvement of bacteria capable of producing glutamine, lysine, arginine, glutamic acid, histidine, proline, isoleucine, etc. illustratively shown in Table 2, the procedure was also effective for strains producing phenylalanine, threonine, valine, ornithine, tryptophane, citrulline, leucine, tyrosine and serine.

The peptide resistance of the thus obtained variants was compared with that of the parent strains.

Onto a liquid medium composed of 0.5 g/dl of glucose, 0.2 g/dl of urea, 0.15 g/dl of ammonium sulfate, 0.3 l g/dl of $KH_2PO_4$, 0.1 g/dl of $K_2HPOO_4$, 0.01 g/dl of $MgSO_4.7H_2O$, 0.1 mg/dl of $CaCl_2.2H_2O$, 100 $\mu$g/l of biotin, 100 $\mu$g/l of thiamine hydrochloride, 0.002 g/dl of $FeSO_4.7H_2O$, 0.002 g/dl of $MnSO_4.7H_2O$ and a peptide in the amount shown in the table and which was adjusted to pH 7.0, there was inoculated a suspension of the cells in sterile water, which had been obtained by culturing in a slant of a natural medium (1 g/dl of peptone, 1 g/dl of yeast extract and 0.5 g/dl of NaCl (pH 7.0) for 24 hours. After culturing for 24 hours, turbidity associated with the growth of bacteria was determined and the growth degree was expressed in terms of relative growth degree (%) (Tables 3 through 8).

5

## Table 2

| Kind of Amino Acid | Parent Strain | Impartion of Peptide Resistance | Strain Having Improved Yield Based On Glucose |
|---|---|---|---|
| Glutamine | Brevibacterium flavum ATCC 14067 | Tyr-Glu | Brevibacterium flavum AJ 12418 (FERM BP-2205 ) |
| | Corynebacterium acetoacidophilum ATCC 13870 | Ala-Glu | Corynebacterium acetoacidophilum AJ 12419 (FERM BP-2206 ) |
| Lysine | Brevibacterium lactofermentum AJ 3445 (FERM P-1944) | Val-Glu | Brevibacterium lactofermentum AJ 12420 (FERM BP-2207 ) |
| | Corynebacterium glutamicum AJ 3399 (FERM P-1615) | Ala-Glu | Corynebacterium glutamicum AJ12421 (FERM BP-2208 ) |
| Arginine | Brevibacterium flavum AJ 3401 (FERM P-1642) | Tyr-Glu | Brevibacterium flavum AJ 12422 (FERM BP-2209 ) |
| Glutamic acid | Brevibacterium lactofermentum ATCC 13869 | Tyr-Glu | Brevibacterium lactofermentum AJ 12423 (FERM BP-2210 ) |
| | Corynebacterium glutamicum ATCC 13032 | Ala-Glu | Corynebacterium glutamicum AJ 12424 (FERM BP-2211 ) |
| Histidine | Brevibacterium flavum AJ 3620 (FERM P-2316) | Trp-Glu | Brevibacterium flavum AJ 12425 (FERM BP-2212 ) |
| | Corynebacterium glutamicum AJ 12092 (FERM P-7273) | Glu-His | Corynebacterium glutamicum AJ 12426 (FERM BP-2213 ) |

| Proline | Brevibacterium flavum AJ 11512 (FERM P-5332) | Tyr-Glu | Brevibacterium flavum AJ 12427 (FERM BP-2214 ) |
|---|---|---|---|
| Isoleucine | Brevibacterium flavum AJ 3686 (FERM P-2433) | Ala-Asp | Brevibacterium flavum AJ 12428 (FERM BP-2215 ) |

## Table 3

Gln

| Peptide / Strain | Tyr-Glu (%) | | | | Ala-Glu (%) | | | |
|---|---|---|---|---|---|---|---|---|
| | 0 | 0.05 | 0.1 | 0.3 | 0 | 0.05 | 0.1 | 0.3 |
| Brevibacterium flavum ATCC 14067 | 100 | 85 | 45 | 0 | | | | |
| Brevibacterium flavum AJ 12418 | 100 | 100 | 100 | 72 | | | | |
| Corynebacterium acetoacidophilum ATCC 13870 | | | | | 100 | 70 | 30 | 0 |
| Corynebacterium acetoacidophilum AJ 12419 | | | | | 100 | 100 | 100 | 65 |

Table 4

Lys*

| Peptide Strain | Val-Glu (%) | | | | Ala-Glu (%) | | | |
|---|---|---|---|---|---|---|---|---|
| | 0 | 0.05 | 0.1 | 0.3 | 0 | 0.05 | 0.1 | 0.3 |
| Brevibacterium lactofermentum ATCC 3445 | 100 | 95 | 80 | 10 | | | | |
| Brevibacterium lactofermentum AJ 12420 | 100 | 100 | 100 | 90 | | | | |
| Corynebacterium glutamicum AJ 3399 (FERM P-1615) | | | | | 100 | 50 | 10 | 0 |
| Corynebacterium glutamicum AJ 12421 | | | | | 100 | 100 | 100 | 100 |

* When Corynebacterium glutamicum was used, 15 mg/dl of methionine was supplemented.

Table 5

Arg*

| Peptide Strain | Tyr-Glu (%) | | | |
|---|---|---|---|---|
| | 0 | 0.05 | 0.1 | 0.3 |
| Brevibacterium flavum AJ 3401 | 100 | 100 | 30 | 0 |
| Brevibacterium flavum AJ 12422 | 100 | 100 | 100 | 95 |

* Liquid medium was supplemented with 5 mg/dl of guanine.

## Table 6

Glu

| Peptide | Tyr-Glu (%) | | | | Ala-Glu (%) | | | |
|---|---|---|---|---|---|---|---|---|
| Strain | 0 | 0.05 | 0.1 | 0.3 | 0 | 0.05 | 0.1 | 0.3 |
| Brevibacterium lactofermentum ATCC 13869 | 100 | 78 | 42 | 0 | | | | |
| Brevibacterium lactofermentum AJ 12423 | 100 | 100 | 95 | 80 | | | | |
| Corynebacterium glutamicum ATCC 13870 | | | | | 100 | 75 | 40 | 0 |
| Corynebacterium glutamicum AJ 12424 | | | | | 100 | 100 | 100 | 94 |

## Table 7

His

| Peptide | Trp-Glu (%) | | | | Glu-His (%) | | | |
|---|---|---|---|---|---|---|---|---|
| Strain | 0 | 0.05 | 0.1 | 0.3 | 0 | 0.05 | 0.1 | 0.3 |
| Brevibacterium flavum AJ 3620 | 100 | 100 | 75 | 36 | | | | |
| Brevibacterium flavum AJ 12425 | 100 | 100 | 100 | 100 | | | | |
| Corynebacterium glutamicum AJ 12092 | | | | | 100 | 100 | 96 | 55 |
| Corynebacterium glutamicum AJ 12426 | | | | | 100 | 100 | 100 | 98 |

## Table 8

**Pro***

| Peptide Strain | Tyr-Glu (%) | | | |
|---|---|---|---|---|
| | 0 | 0.05 | 0.1 | 0.3 |
| Brevibacterium flavum AJ 11512 | 100 | 90 | 30 | 0 |
| Brevibacterium flavum AJ 12427 | 100 | 100 | 95 | 60 |

\* Liquid medium was supplemented with 15 mg/dl of isoleucine.

## Table 9

**Ile**

| Peptide Strain | Ala-Asp (%) | | | |
|---|---|---|---|---|
| | 0 | 0.05 | 0.1 | 0.3 |
| Brevibacterium flavum AJ 3686 | 100 | 70 | 20 | 0 |
| Brevibacterium flavum AJ 12428 | 100 | 100 | 98 | 70 |

Media used for culturing such variants are conventional media containing carbon sources, nitrogen sources, inorganic ions, substances satisfying nutrient auxotrophy and, if necessary, other organic trace nutrients including vitamins, etc. As carbon sources, there are preferably used carbohydrates such as glucose, sucrose, etc., organic acids such as acetic acid, etc. As nitrogen sources, there are preferably used aqueous ammonia, ammonia gas, ammonium salts, etc. As inorganic ions, potassium ions, sodium ions, magnesium ions, phosphate ions, and the like are appropriately added to media, depending upon necessity. Incubation is preferably conducted under aerobic conditions. When the incubation is carried out while adjusting the pH of the medium to a range from 4 to 8 at a temperature of from 25 °C to 37 °C, more preferable results can be obtained. Thus, during a cultivation for 1 to 7 days, remarkable amounts of L-amino acid are produced and accumulated in the media. Subsequently using a separation method, such as ion exchange etc., crystals of L-amino acid can be obtained.

The present invention is further described with reference to the following examples.

Example 1

300 ml aliquots of an aqueous solution medium, comprising 10 % of glucose, 1 % of ammonium sulfate, 0.25 % of potassium primary phosphate, 0.04 % of magnesium sulfate, 0.001 % of ferrous sulfate, 350 $\mu$g/l of thiamine hydrochloride, 5 $\mu$g/l of biotin and 0.5 ml/dl of Aji-Eki (soybean protein hydrolysate) (registered trademark), pH 7.0, were separately charged in small sized glass jar fermenters. After sterilizing in a conventional manner, various L-glutamine-producing bacteria strains shown in Table 10, which had been previously grown on bouillon slants at 30 °C for 24 hours, were inoculated thereon. Then, incubation was carried out at 31.5 °C for 30 hours at 1200 rpm with aeration in an amount of 1/4 volume per minute, while keeping the pH to 6.5 with ammonia gas. The yield of L-glutamine produced and accumulated in the solution after completion of the fermentation based on glucose is shown in Table 10.

Table 10

| Strain | Property | Yield of L-Glutamine Based on Glucose (%) |
|---|---|---|
| Brevibacterium flavum ATCC 14067 (AJ 11576) | wild | 29.0 |
| Brevibacterium flavum AJ 12418 | Imparted with Tyr-Glu resistance | 40.0 |
| Corynebacterium acetoacidophilum ATCC 13870 | wild | 22.5 |
| Corynebacterium acetoacidophilum AJ 12419 | Imparted with Ala-Glu resistance | 34.0 |

After completion of the fermentation using Brevibacterium flavum AJ 12418 the cells were removed by centrifugation from 1 liter of the solution obtained to give a supernatant. From the supernatant, L-glutamine was isolated in a conventional manner using a ion exchange resin to give 19.0 g of L-glutamine as crystals.

Example 2

300 ml aliquots of an aqueous solution medium composed of 10 % of glucose, 2 % of ammonium sulfate, 0.1 % of potassium primary phosphate, 0.04 % of magnesium sulfate, 0.001 % of ferrous sulfate, 200 $\mu$g/l of thiamine hydrochloride, 500 $\mu$g/l of biotin, 5 ml/dl of Aji-Eki (registered trademark), 1 mg/dl of nicotinamide and 0.1 % of DL-alanine, pH 7.0, were separately charged in small sized glass jar fermenters. After sterilizing in a conventional manner, various L-lysine-producing bacteria strains shown in Table 11, which had been previously grown on bouillon slants at 30 °C for 48 hours, were inoculated theron. Then, incubation was carried out at 31.5 °C for 48 hours at 1200 rpm with aeration in a rate of 1/2 volume per minute, while keeping the pH at 7.0 with ammonia gas. The yield of L-lysine produced and accumulated in the solution after completion of the fermentation based on glucose is shown in Table 11.

Table 11

| Strain | Property | Yield of L-Lysine Based on Glucose (%) |
|---|---|---|
| Brevibacterium lactofermentum AJ 3445(US-A-3 929 571) | AEC [(S-(2-aminoethyl)-L-cysteine] resistance | 16.0 |
| Brevibacterium lactofermentum AJ 12420 | Imparted with Val-Glu resistance | 31.0 |
| Corynebacterium glutamicum AJ 3399 (JP-A-48891/1974) | Met, AEC resistance | 23.0 |
| Corynebacterium glutamicum AJ 12421 | Imparted with Ala-Glu resistance | 32.0 |

The cells were removed from 1 liter of the solution obtained after completion of the fermentation using Brevibacterium lactofermentum AJ 12420 by centrifugation to give a supernatant. From the supernatant, L-lysine was isolated in a conventional manner using an ion exchange resin to give 19.2 g of L-lysine as crystals.

Example 3

300 ml aliquots of an aqueous solution medium composed of 10 % of glucose, 4 % of ammonium sulfate, 0.1 % of potassium primary phosphate, 0.04 % of magnesium sulfate, 0.001 % of ferrous sulfate, 0.001 % of manganese sulfate, 100 $\mu$g/l of thiamine hydrochloride, 100 $\mu$g/l of biotin, 5 ml/dl of Aji-Eki (registered trademark) and 0.2 % of yeast extract, pH 7.0, were separately charged in small sized glass jar fermenters. After sterilizing in a conventional manner, various L-arginine-producing bacteria strains shown in Table 12, which had been previously grown on bouillon slants at 30 °C for 24 hours, were inoculated thereon. Then, incubation was carried out at 31.5 °C for 48 hours at 1200 rpm with an aeration rate of 1/2 volume per minute, while keeping the pH to 7.0 with ammonia gas. The yield of L-arginine produced and accumulated in the solution after completion of the fermentation based on glucose is shown in Table 12.

Table 12

| Strain | Property | Yield of L-Arginine Based on Glucose (%) |
|---|---|---|
| Brevibacterium flavum AJ 3401 (US-A-3 878 044) (FERM P-1639) | Gua$^-$(requirement of guanine), 2-thiazolyl-alanine resistance | 25.5 |
| Brevibacterium flavum AJ 12422 (FERM BP-2209 ) | Imparted with Tyr-Glu resistance | 32.0 |

The cells were removed by centrifugation from 1 liter of the solution obtained after completion of the fermentation using Brevibacterium flavum AJ 12422 to give a supernatant. From the supernatant, L-arginine was isolated in a conventional manner using an ion exchange resin to give 17.3 g of L-arginine as crystals.

Example 4

300 ml aliquots of an aqueous solution medium comprising 10 % of glucose, 1 % of ammonium sulfate, 0.2 % of potassium primary phosphate, 0.1 % of magnesium sulfate, 0.001 % of ferrous sulfate, 0.001 % of manganese sulfate, 500 μg/l of thiamine hydrochloride, 5 μg/dl of biotin and 1 ml/dl of Aji-Eki (registered trademark), pH 7.2, were separately charged in small sized glass jar fermenters. After sterilizing in an autoclave, various L-glutamic acid-producing bacteria strains shown in Table 13, which had been previously grown on bouillon slants at 30 °C for 24 hours, were inoculated thereon. Then, incubation was carried out at 31.5 °C for 48 hours at 1200 rpm with an aeration rate of 1/2 volume per minute, while keeping the pH at 7.2 with ammonia gas. The yield of L-glutamic acid produced and accumulated in the solution after completion of the fermentation based on glucose is shown in Table 13.

Table 13

| Strain | Property | Yield of L-Glutami acid Based on Glucose (%) |
|---|---|---|
| Brevibacterium lactofermentum ATCC 13869 | wild strain | 44.2 |
| Brevibacterium lactofermentum AJ 12423 | Imparted with Tyr-Glu resistance | 49.5 |
| Corynebacterium glutamicum ATCC 13032 | wild strain | 40.1 |
| Corynebacterium glutamicum AJ 12424 | Imparted with Ala-Glu resistance | 46.8 |

The cells were removed by centrifugation from 1 liter of the solution obtained after completion of the fermentation using Brevibacterium lactofermentum AJ 12423 to give the supernatant. From the supernatant, L-glutamic acid was isolated in a conventional manner using ion exchange resin to give 35.5 g of L-glutamic acid as crystals.

Example 5

300 ml aliquots of an aqueous solution medium comprising 10 % of glucose, 0.5 % of ammonium sulfate, 0.15 % of potassium primary phosphate, 0.1 % of magnesium sulfate, 0.001 % of ferrous sulfate, 0.001 % of manganese sulfate, 300 μg/l of thiamine hydrochloride, 350 μg/l of biotin, 5 ml/dl of Aji-Eki (registered trademark) and 0.5 % of ammonium acetate, pH 7.0, were separately charged in small sized glass jar fermenters. After sterilizing in an autoclave, various L-histidine-producing bacteria strains shown in Table 14, which had been previously grown on bouillon slants at 30 °C for 24 hours, were inoculated thereon. Then, incubation was carried out at 31.5 °C for 48 hours at 1200 rpm with an aeration rate of 1/2 volume per minute, while keeping the pH at 6.5 with ammonia gas. The yield of L-histidine produced and accumulated in the solution after completion of the fermentation based on glucose is shown in Table 14.

Table 14

| Strain | Property | Yield of L-Histidine Based on Glucose (%) |
|---|---|---|
| Brevibacterium flavum AJ 3620 (JP-A-70591/1975) | 2-AT(2-thiazole alanine), sulfadiazine, cobalamine resistance | 7.2 |
| Brevibacterium flavum AJ 12425 | Imparted with Trp-Glu resistance | 10.0 |
| Corynebacterium glutamicum AJ 12092 (JP-A-36197/1987) | 2-AT(2-thiazole alanine) resistance | 5.0 |
| Corynebacterium glutamicum AJ 12426 | Imparted with Glu-His resistance | 9.3 |

The cells were removed by centrifugation from 1 liter of the solution obtained after completion of the fermentation using Corynebacterium glutamicum AJ 12425 to give a supernatant. From the supernatant, L-histidine was isolated in a conventional manner using ion exchange resin to give 4.7 g of L-histidine as crystals.

Example 6

300 ml aliquots of an aqueous solution medium comprising 10 % of glucose, 4 % of ammonium sulfate, 0.1 % of potassium primary phosphate, 0.5 % of magnesium sulfate, 0.001 % of ferrous sulfate, 0.001 % of manganese sulfate, 100 μg/l of thiamine hydrochloride, 350 μg/l of biotin, 1 ml/dl of Aji-Eki (registered trademark) and 35 mg/dl of L-isoleucine, pH 7.0, were separately charged in small sized glass far fermenters. After sterilizing in a conventional manner, various L-proline-producing bacteria strains shown in Table 15, which had been previously grown on bouillon slants at 30 °C for 24 hours, were inoculated thereon. Then, incubation was carried out at 31.5 °C for 48 hours at 1200 rpm with an aeration rate of 1/2 volume per minute, while keeping the pH at 7.0 with ammonia gas. After completion of the fermentation, the yield of L-proline produced and accumulated in the solution based on glucose is shown in Table 15.

Table 15

| Strain | Property | Yield of L-Proline Based on Glucose (%) |
|---|---|---|
| Brevibacterium flavum AJ 11512 (JP-A-2691/1982) | Ile⁻, sulfaguanidine resistance | 21.0 |
| Brevibacterium flavum AJ 12427 | Imparted with Tyr-Glu resistance | 29.0 |

The cells were removed from 1 liter of the solution obtained after completion of the fermentation using Brevibacterium flavum AJ 12 427 by centrifugation to give a supernatant. From the supernatant, L-proline was isolated in a conventional manner using ion exchange resin to give 17.5 g of L-proline as crystals.

Example 7

300 ml aliquots of an aqueous solution medium composed of 10 % of glucose, 1 % of ammonium sulfate, 0.1 % of potassium primary phosphate, 0.04 % of magnesium sulfate, 0.001 % of ferrous sulfate, 0.001 % of manganese sulfate, 100 μg/l of thiamine hydrochloride, 100 μg/l of biotin and 2 ml/dl of Aji-Eki (registered trademark), pH 7.0 were separately charged in small sized glass jar fermenters. After sterilizing in a conventional manner, various L-isoleucine-producing bacteria strains shown in Table 16, which had

13

been previously grown on bouillon slants at 30 °C for 24 hours, were inoculated thereon. Then, incubation was carried out at 31.5 °C for 48 hours at 1200 rpm with an aeration rate of 1/2 volume per minute, while keeping the pH at 7.3 with ammonia gas. The yield of L-isoleucine produced and accumulated in the solution after completion of the fermentation based on glucose is shown in Table 16.

Table 16

| Strain | Property | Yield of L-Isoleucine Based on Glucose (%) |
|---|---|---|
| Brevibacterium flavum AJ 3686 (US-A-4 656 135) | AHV ($\alpha$-amino-$\beta$-hydroxyvaleric acid) resistance | 8.5 |
| Brevibacterium flavum AJ 12428 | Imparted with Ala-Asp resistance | 13.0 |

The cells were removed from 1 liter of the solution obtained after completion of the fermentation using Brevibacterium flavum AJ 12428 by centrifugation to give the supernatant. From the supernatant, L-isoleucine was isolated in a conventional manner using a ion exchange resin to give 6.5 g of L-isoleucine as crystals.

According to the present invention, various L-amino acids can be obtained in a good yield.

## Claims

1. A process for producing an L-amino acid which comprises culturing in a liquid medium an L-amino acid-producing microorganism belonging to the genus Brevibacterium or the genus Corynebacterium, accumulating said L-amino acid in the culture medium and collecting said L-amino acid from the culture medium, wherein the L-amino acid-producing microorganism has resistance to a peptide containing glutamic acid or aspartic acid.

2. A process for producing an L-amino acid according to claim 1, wherein said peptide containing glutamic acid or aspartic acid is X-Glu, Glu-X, X-Asp or Asp-X;
   wherein X represents

```
Gly, Ala, Val, Leu, Ile, Arg,
Asp, Cit, Cys, Lys, Glu, Gln, His, Met, Orn, Pro, OH-Pro,
Phe, Tyr, Trp, Ser or Thr.
```

3. A microorganism belonging to the genus Brevibacterium or the genus Corynebacterium which is Brevibacterium flavum AJ 12418 (FERM BP-2205), Corynebacterium acetoacidophilum AJ 12419 (FERM BP-2206), Brevibacterium lactofermentum AJ 12420 (FERM BP-2207), Corynebacterium glutamicum AJ 12421 (FERM BP-2208), Brevibacterium flavum AJ 12422 (FERM BP-2209), Brevibacterium lactofermentum AJ 12423 (FERM BP-2210), Corynebacterium glutamicum AJ 12424 (FERM BP-2211), Brevibacterium flavum AJ 12425 (FERM BP-2212), Corynebacterium glutamicum AJ 12426 (FERM BP-2213), Brevibacterium flavum AJ 12427 (FERM BP-2214), Brevibacterium flavum AJ 12428 ((FERM BP-2215).

## Patentansprüche

1. Verfahren zur Herstellung von L-Aminosäuren, welches umfaßt :
   Züchten eines eine L-Aminosäure produzierenden Mikroorganismus, welcher der Gattung Brevibacterium oder der Gattung Corynebacterium angehört, in einem flüssigen Medium,
   Anreichern der L-Aminosäure in dem Kulturmedium, und
   Gewinnen der L-Aminosäure aus dem Kulturmedium, wobei der L-Aminosäure produzierende Mikroorganismus gegen ein Glutaminsäure oder Asparaginsäure enthaltendes Peptid resistent ist.

2. Verfahren zur Synthese von L-Aminosäuren nach Anspruch 1, wobei das Glutaminsäure oder Asparaginsäure enthaltende Peptid X-Glu, Glu-X, X-Asp oder Asp-X ist, wobei X

```
Gly, Ala, Val, Leu, Ile, Arg, Asp, Cit, Cys, Lys, Glu, Gln,
His, Met, Orn, Pro, OH-Pro, Phe, Tyr, Trp, Ser oder Thr
```

ist.

3. Mikroorganismus, welcher der Gattung Brevibacterium oder der Gattung Corynebacterium angehört, der Brevibacterium flavum AJ 12418 (FERM BP-2205), Corynebacterium acetoacidophilum AJ 12419 (FERM BP-2206), Brevibacterium lactofermentum AJ 12420 (FERM BP-2207), Corynebacterium glutamicum AJ 12421 (FERM BP-2208), Brevibacterium flavum AJ 12422 (FERM BP-2209), Brevibacterium lactofermentum AJ 12423 (FERM BP-2210), Corynebacterium glutamicum AJ 12424 (FERM BP-2211), Brevibacterium flavum AJ 12425 (FERM BP-2212), Corynebacterium glutamicum AJ 12426 (FERM BP-2213), Brevibacterium flavum AJ 12427 (FERM BP-2214), Brevibacterium flavum AJ 12428 (FERM BP-2215) ist.

**Revendications**

1. Procédé de production d'un L-aminoacide, qui comprend la culture dans un milieu liquide d'un microorganisme producteur d'un L-aminoacide appartenant au genre Brevibacterium ou au genre Corynebacterium, l'accumulation dudit L-aminoacide dans le milieu de culture et la récolte dudit L-aminoacide à partir du milieu de culture, le microorganisme producteur du L-aminoacide ayant une résistance à un peptide contenant de l'acide glutamique ou de l'acide aspartique.

2. Procédé de production d'un L-aminoacide selon la revendication 1, dans lequel ledit peptide contenant l'acide glutamique ou l'acide aspartique est X-Glu, Glu-X, X-Asp ou Asp-X, où X représente

Gly, Ala, Val, Leu, Ile, Arg, Asp, Cit, Cys, Lys, Glu, Gln, His, Met, Orn, Pro, OH–Pro, Phe, Tyr, Trp, Ser ou Thr.

3. Microorganisme appartenant au genre Brevibacterium ou Corynebacterium, qui est Brevibacterium flavum AJ 12418 (FERM BP-2205), Corynebacterium acetoacidophilum AJ 12419 (FERM BP-2206), Brevibacterium lactofermentum AJ 12420 (FERM BP-2207), Corynebacterium glutamicum AJ 12421 (FERM BP-2208), Brevibacterium flavum AJ 12422 (FERM BP-2209), Brevibacterium lactofermentum AJ 12423 (FERM BP-2210), Corynebacterium glutamicum AJ 12424 (FERM BP-2211), Brevibacterium flavum AJ 12425 (FERM BP-2212), Corynebacterium glutamicum AJ 12426 (FERM BP-2213), Brevibacterium flavum AJ 12427 (FERM BP-2214), Brevibacterium flavum AJ 12428 (FERM BP-2215).